# EUROPEAN PATENT SPECIFICATION

(11) **EP 2 703 013 B1**
(45) Date of publication and mention of the grant of the patent: **19.10.2016**
(21) Application number: 12182621.8
(22) Date of filing: 31.08.2012
(51) Int. Cl.: A61L 2/18, A61L 2/26, B67D 1/00, B01F 15/00

(54) **Sterilizing liquid dispenser**
Sterilflüssigkeitsspender
Distributeur de liquide de stérilisation

(43) Date of publication of application: 05.03.2014
(73) Proprietor: Tristel PLC, Snailwell Newmarket, Cambridgeshire CB8 7NY (GB)
(72) Inventor: Secker, Alan Frederick, Swaffham PE37 7BZ (GB); Stallwood, Wayne, Bury St. Edmunds IP33 3LG (GB); Swinney, Paul, Newmarket, CB8 7NY (GB)
(74) Representative: Dummett Copp LLP

(56) References cited:
- EP-A1- 1 669 322
- WO-A1-2005/011759
- CA-A1- 2 052 829
- US-A1- 2009 188 938

## Description

### BACKGROUND

### a. Field of the Invention

The present invention relates to a sterilizing liquid dispenser and particularly to a sterilizing liquid dispenser for preparing and dispensing batches of sterilizing liquid from multi-part reagents as and when required. Aqueous chlorine dioxide (ClO₂) is a particularly preferred sterilizing liquid.

### b. Related Art

Batches of aqueous ClO₂ sterilizing liquids are made by a user adding two or more reagents to water in a jug or bucket. The reagents react to produce ClO₂. The resultant mixture may be used to sterilize surfaces or added to sterilizing apparatus such as the Stella® tray described in WO2008/020770. Suitable reagents are well known to those skilled in the art and include chlorite and acid; chlorate, peroxide and acid; and chlorite, hypochlorite, and a suitable buffer.

Apparatus for generating and dispensing a stream of aqueous ClO₂ for use in medical facilities is described in WO 2005/011759. Fluid portions of a first reagent and a second reagent are pumped into the proximal end of an elongate reaction chamber, the reagents being selected to react together to form aqueous ClO₂. A stream of water is provided around or adjacent to the distal end of the reaction chamber. Continued pumping of the reagents causes the reagent mixture to travel through the reaction chamber and then into the stream of water to form a stream of aqueous ClO₂ sterilizing liquid.

The pumping rates and the internal dimensions of the reaction chamber are selected so that the reaction to form ClO₂ is substantially complete when the reagent mixture exits the reaction chamber. The accurate pumping requires use of a diaphragm pump or a piston pump *via* a pressure control valve so that each fluid portion is pumped at substantially constant pressure.

EP 1 669 322 A1 discloses a dispenser with two or more reservoirs, each having an outlet valve, all valves being actuated in common. The reservoirs do not have any inlet valves so there is no metering possible by closing/opening the inlet/outlet valves.

### SUMMARY OF THE INVENTION

Aspects of the invention are specified in the independent claims. Preferred features are specified in the dependent claims.

In one embodiment, the invention provides a gravity-fed system in which a measured amount of each part of a multi-part sterilant chemistry can be delivered into a controlled volume of water in a single operation, to form a sterilizing fluid. In particular each part of the multi part chemistry can be dispensed independently, but at the same time, by a user of the system.

In a preferred embodiment, the dispenser operates automatically with a single button press. The dispenser may operate without the use of a motor or any power source. Preferably the dispenser is connected to a supply of mains water such that a mixing tank is only filled immediately prior to discharge of the resulting sterilizing fluid, thereby reducing or eliminating the risks associated with a supply of standing water.

### BRIEF DESCRIPTION OF THE DRAWINGS

The invention will now be further described, by way of example only, with reference to the following drawings in which:

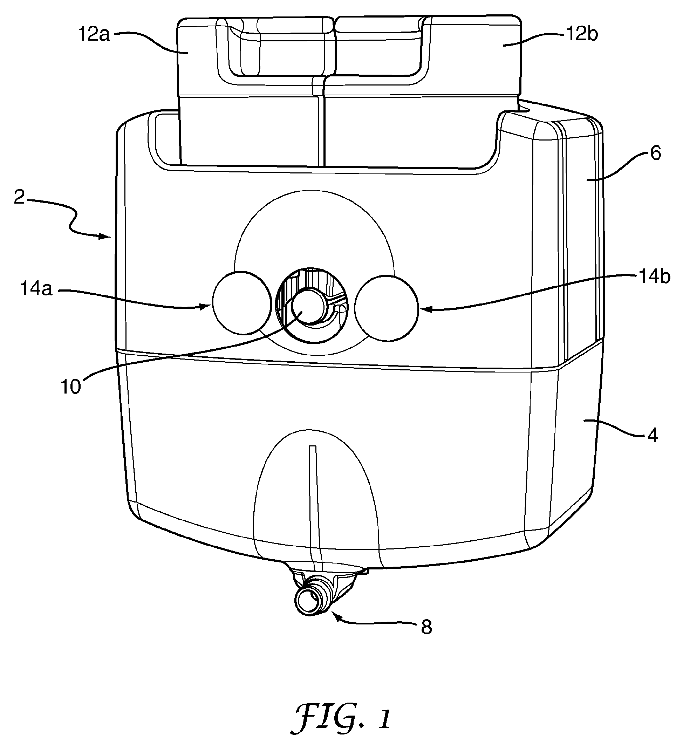

Figure 1 shows a sterilizing liquid dispenser in accordance with a first embodiment of the present invention;
Figure 2 is a perspective view showing cartridges for use in the dispenser of Figure 1;
Figure 3 shows the dispenser of Figure 1 with the cartridges removed;
Figure 4 shows the dispenser of Figure 3 with the top cover removed;
Figures 5 and 6 are perspective views of the reservoir unit of Figure 4 from above and below;
Figures 7 and 8 are views showing spatial relationships between various components of the dispenser of Figure 1;
Figure 9 shows the actuator of the dispenser of Figure 1;
Figure 10 shows parts of the dispenser of Figure 1 with top cover and mixing tank removed;
Figures 11 and 12 are part sectional views of the dispenser of Figure 10 showing the actuator in STANDBY and ACTIVE modes respectively;
Figures 13 and 14 are simplified closer views corresponding to Figures 11 and 12 respectively;
Figure 15 illustrates the dispenser of Figure 1 in use;
Figure 16 shows a sterilizing liquid dispenser in accordance with a second embodiment of the present invention in use and connected to a suitable sterilizing fluid receptacle; and
Figure 17 shows part of the actuating mechanism of the dispenser of Figure 16.

### DETAILED DESCRIPTION

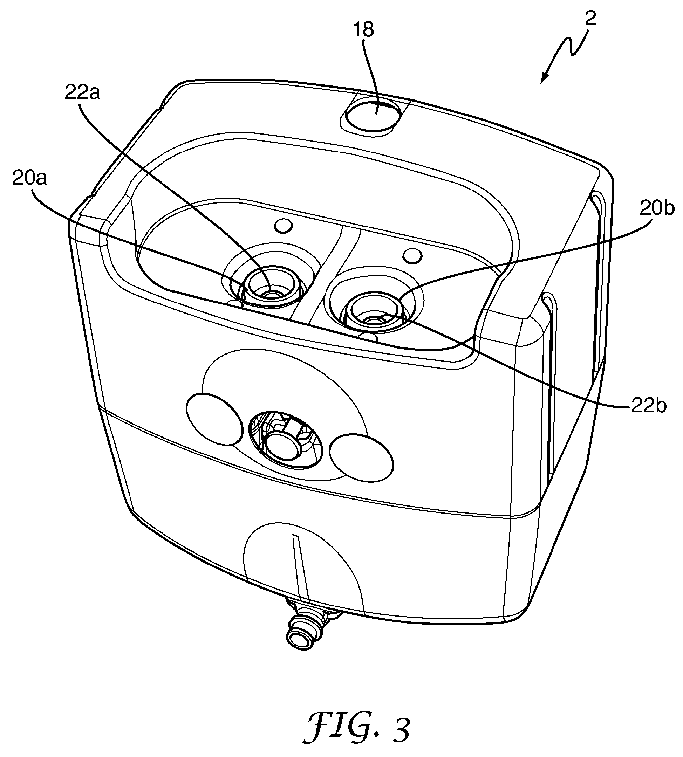

A first exemplified sterilizing liquid dispenser 2 comprises a mixing tank 4 having an outlet 8. A top cover 6 houses a user-operable actuator with a push-button 10. A first cartridge 12a contains a first fluid reagent, and a second cartridge 12b contains a second fluid reagent. The first fluid reagent and the second fluid reagent react when mixed to provide an aqueous chlorine dioxide sterilizing liquid. Windows 14a, 14b in the top cover allow a user to view fluid levels within the dispenser 2. A port 18 (Figure 3) in the top cover is provided for a water inlet.

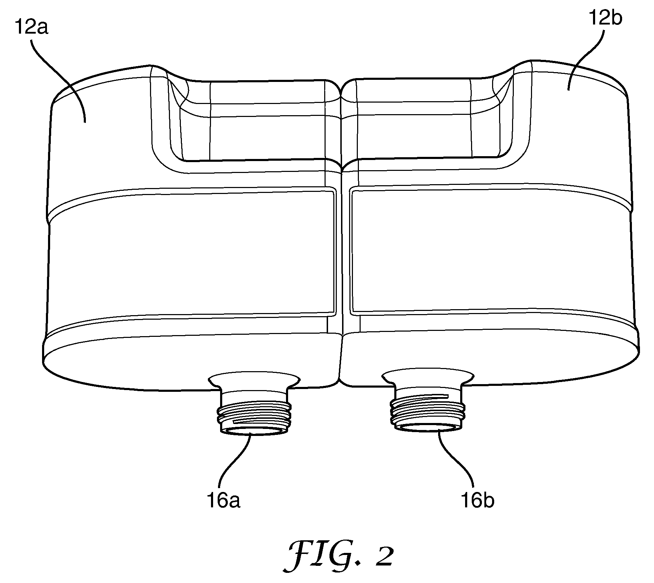

Referring to Figures 2 and 3, each cartridge 12a, 12b has a spout 16a, 16b which locates in a corresponding cartridge coupling or port 20a, 20b. Each cartridge coupling 20a, 20b houses a corresponding cutting member 22a, 22b. The cartridges 12 are supplied with the spouts 16 sealed by a frangible seal. When a spout 16 is fully inserted in the corresponding coupling 20, the cutting member 22 breaks the seal, allowing the fluid reagent to flow out under valve control as will be described below.

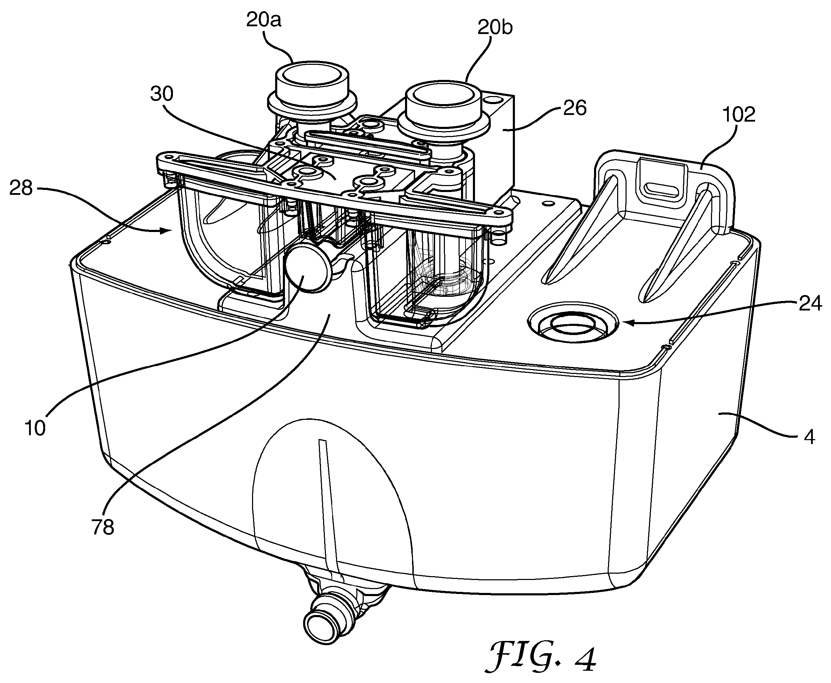

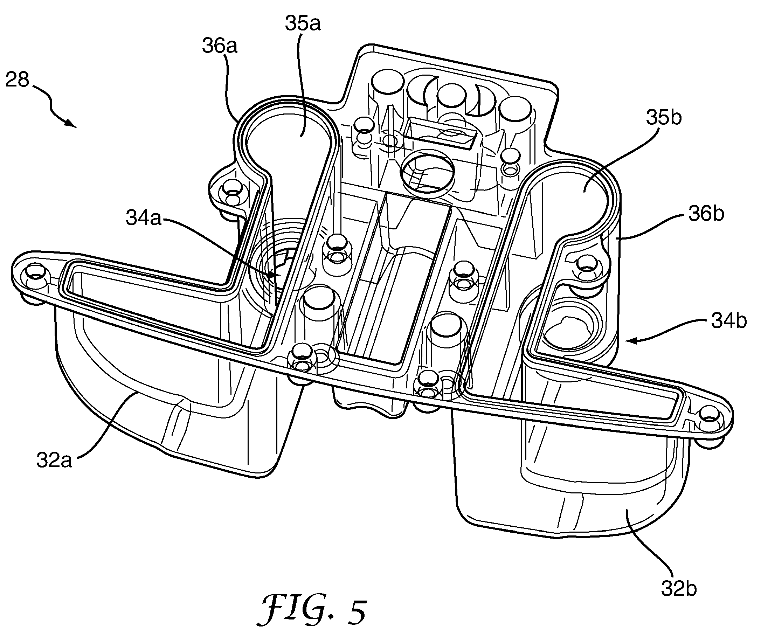

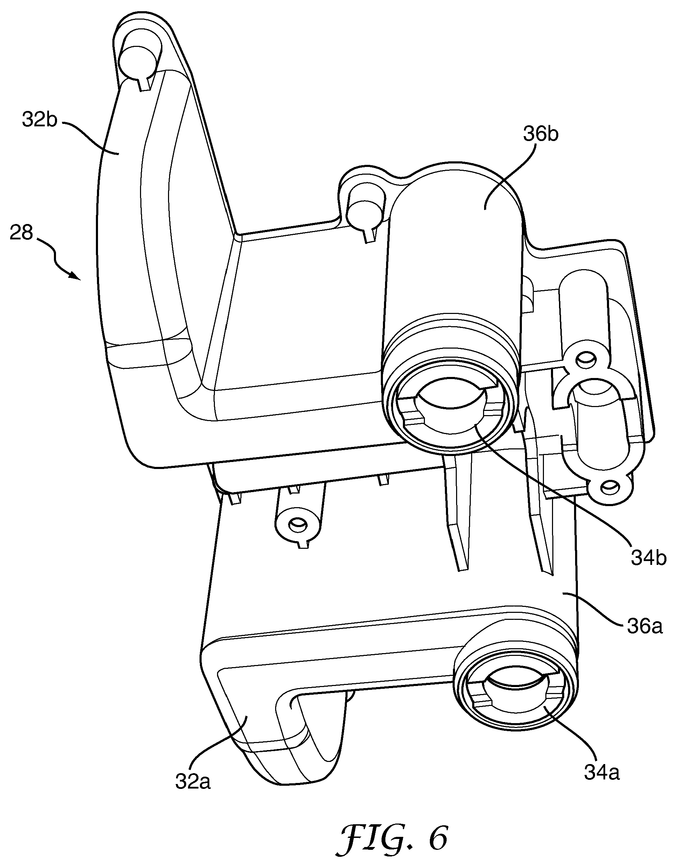

The mixing tank 4 has a cover 78 (Figure 4) which is mounted over an opening in a top wall of the mixing tank 4. Components forming an actuating or dispensing mechanism for dispensing the fluid reagents and controlling flow of water into the mixing tank are mounted on the cover 78. The components include a reservoir unit 28 (best shown in Figures 5 and 6) with a reservoir top cover 30, and a valve housing 26 containing a valve mechanism for controlling input of water to the mixing tank 4. A bulls-eye level 24 in a top wall of the tank 4 allows a user to ensure that the dispenser 2 is level mounted. The dispenser 2 may be wall mounted by means of brackets 102, one of which is visible in Figure 4.

The reservoir unit 28 in this example is symmetric about a central axis and includes a first reservoir 32a and a second reservoir 32b for receiving first and second fluid reagents respectively from the first cartridge 12a and the second cartridge 12b. The reservoir unit 28 is formed from a translucent plastics material so that a user can see through the windows 14 to view fluid levels in the reservoirs 32. Each reservoir 32a, 32b includes a corresponding integrally formed shuttle valve housing 36a, 36b having an outlet 34a, 34b in fluid connection to the mixing tank 4 and an inlet 35a, 35b in fluid connection with a respective cartridge coupling 20a, 20b.

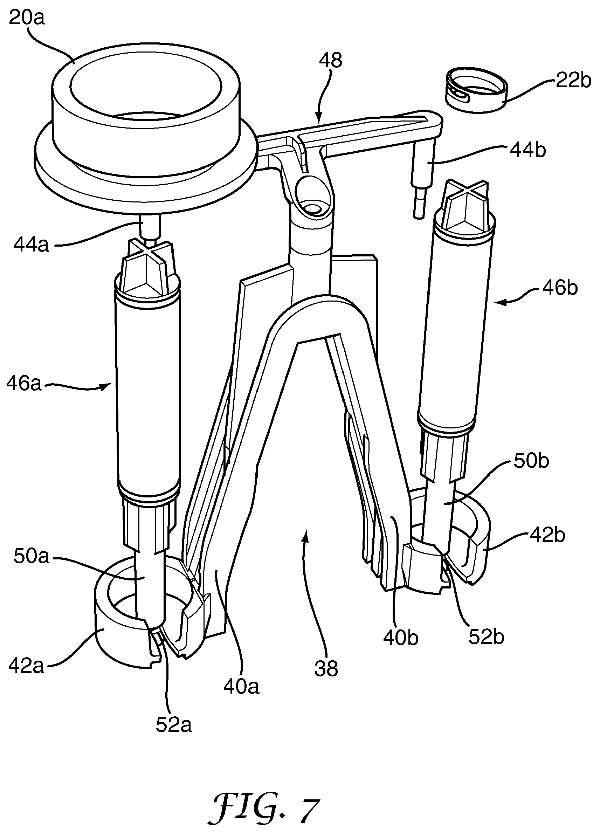

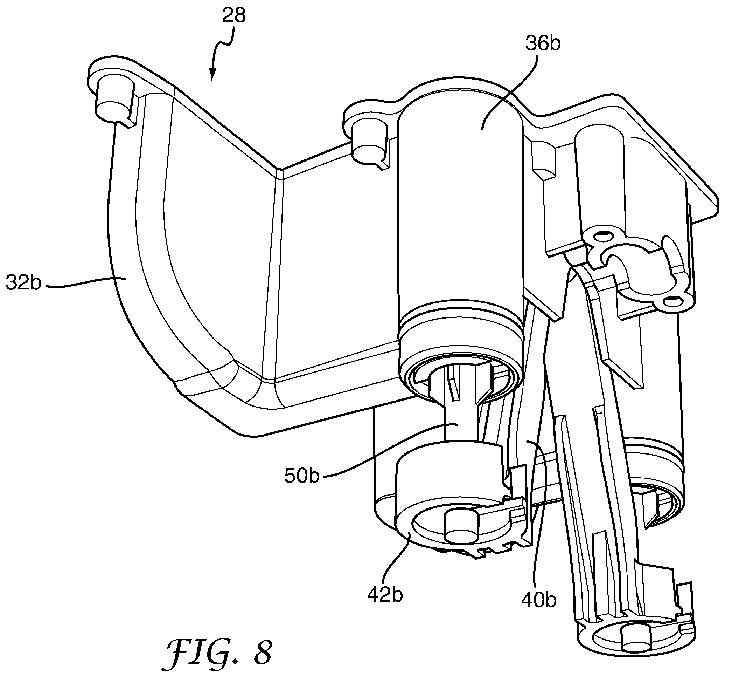

Referring now to Figures 7 and 8, a reservoir valve, in this example, a shuttle valve, 46a, 46b is housed in each corresponding housing 36a, 36b. When the shuttle valve 46 is in an upper position at the top of the housing 36 it provides a fluid seal between the cartridge coupling 20 and the reservoir 32, and it leaves open the reservoir outlet 34. In this configuration, liquid in the reservoirs 32 will drain under gravity through the outlet 34 into the mixing tank 4. When the shuttle valve 46 is in a lower position at the bottom of the housing 36 it seals the reservoir outlet 34, preventing flow of liquid from the reservoir 32 into the mixing tank 4, and opens the seal between the cartridge coupling 20 and the reservoir 32. In this configuration, reagent liquid will drain from the cartridge 12 into the reservoir 32. In this way, the upper end of the shuttle valve acts as a reservoir inlet valve and the lower end of the shuttle valve acts as a reservoir outlet valve. Each valve mechanism is similar to that of a drinks dispenser for dispensing metered quantities of spirits. A ventilation unit 48 provides ventilation tubes 44a 44b which prevent vacuum formation or airlocks within the valve housings 36.

In other embodiments, the reservoir valve may comprise separate but linked inlet and outlet valves. In these arrangements the reservoir inlet valves are located in the reservoir inlet 35 and provide a movable seal between the cartridge coupling 20 and the reservoir 32 and the reservoir outlet valves are located within the reservoir outlet 34 and provide a movable seal between the reservoir 32 and the mixing tank 4.

A dose arm unit 38 includes a first dose arm 40a and a second dose arm 40b. The free end of each arm 40a, 40b is provided with a corresponding radially slotted cup 42a, 42b. Each shuttle valve 46 has a foot member 50 which includes a circumferential groove 52. Each groove 52 is located in a slot in a cup 42 so that each shuttle valve 46 is coupled to a dose arm 40. Upward or downward movement of the dose arm unit 38 will cause corresponding movement of the shuttle valves 46 within the shuttle valve housings 36. In a preferred embodiment, the dose arm unit 38 is arranged such that the shuttle valves 46a, 46b move simultaneously due to movement of the dose arm unit. In some embodiments, each dose arm 40a, 40b may be connected to both an inlet valve and an outlet valve, or alternatively may be connected to a linking member connecting the inlet and outlet valves.

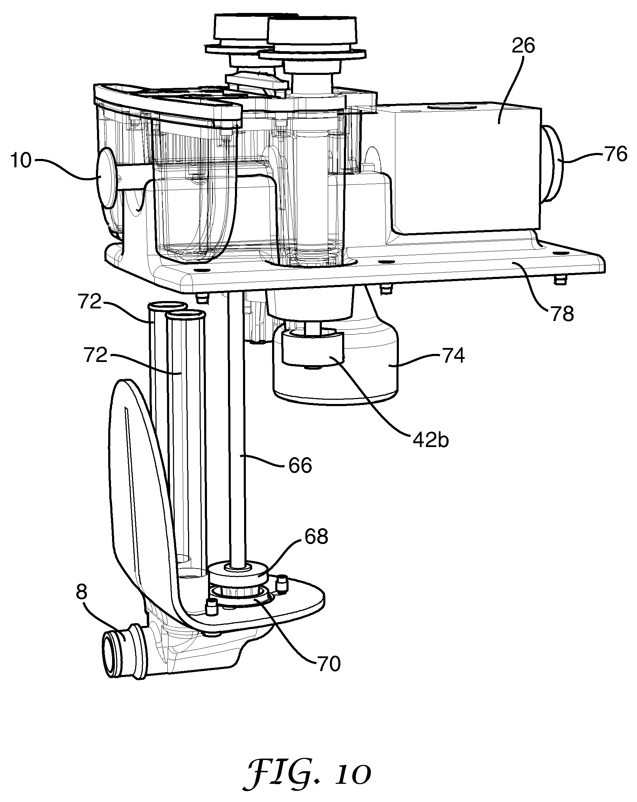

With reference to Figure 10, egress of liquid from the mixing tank 4 is controlled by a washer 68 on a drain control arm 66. When the drain control arm 66 is lowered, it brings the washer 68 into sealing engagement with a drain outlet flange 70, thereby preventing fluid exit via the outlet 8. When the drain control arm 66 is raised, the washer 68 is lifted from the drain outlet flange 70, allowing liquid to drain from the tank via the outlet 8. Overflow pipes 72 are provided in fluid connection with the outlet 8 to permit automatic drainage in the event that the liquid level in the tank exceeds a predetermined maximum. A shut-off float 74 is provided to prevent the maximum liquid level being exceeded, as will be discussed in more detail presently.

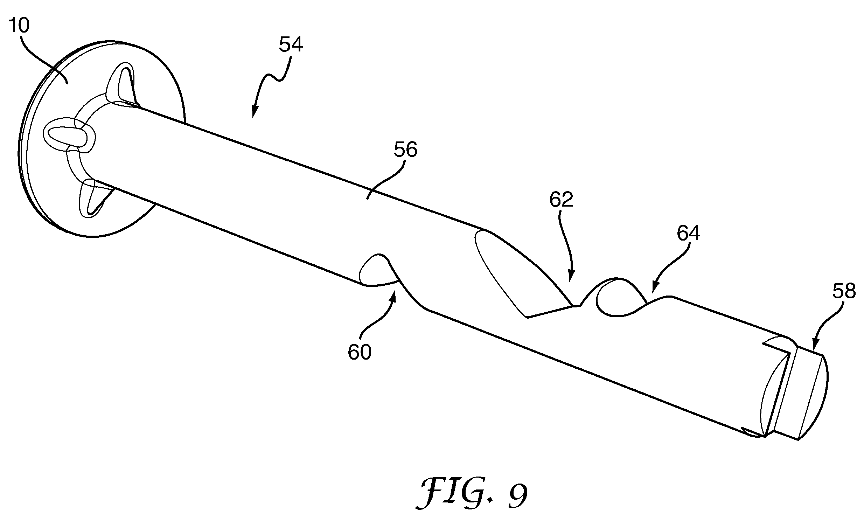

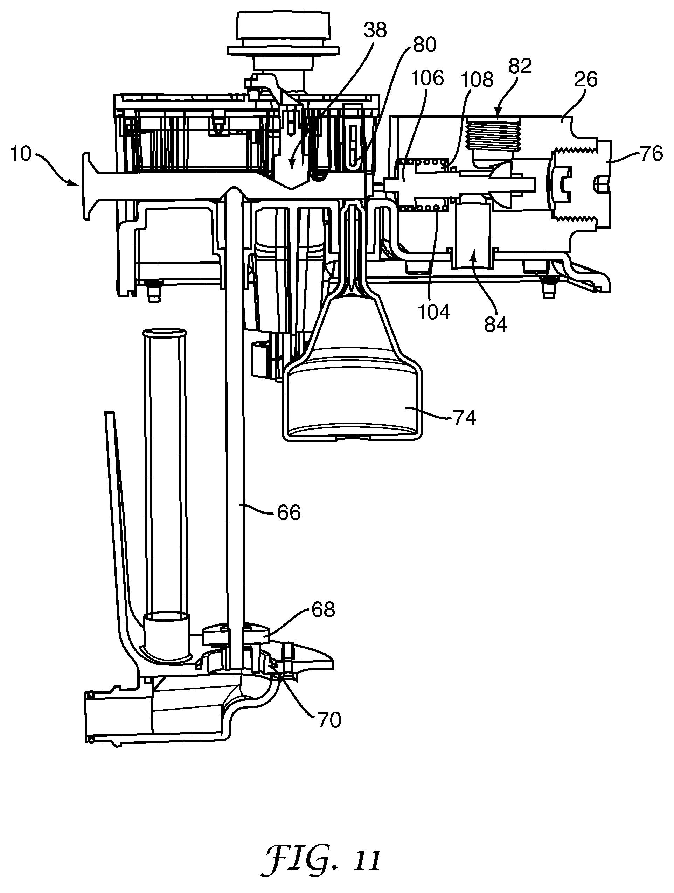

In this example, the actuator 54 (Figure 9) is a push-rod having a shaft 56 with a button 10 at its proximal end and a structure 58 at its distal end for engagement with a valve stem. A first notch 60 provides cam surfaces for engagement with corresponding surfaces on the drain control arm 66, and a second notch 62 provides cam surfaces for engagement with corresponding surfaces on the dose arm unit 38. The shaft 56 also has a groove 64 for receiving a shut-off float arm 80 which is integrally formed with the shut-off float 74, as shown in Figures 11-14 which are described below.

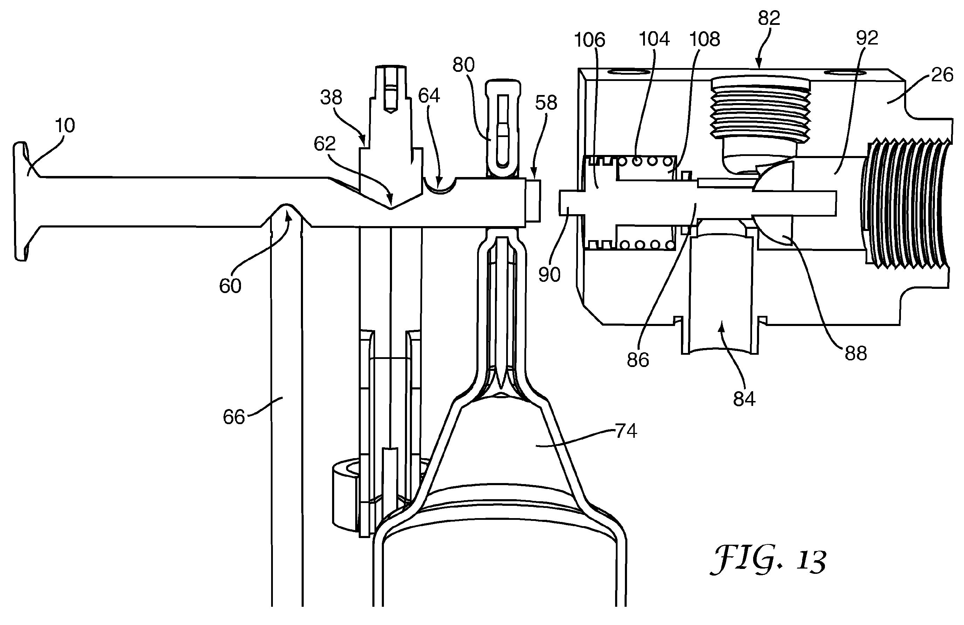

The valve housing 26 has a water inlet 82 for receiving an inlet hose (for example a standard braided washing machine hose) via the opening 18 in the top cover, and a water outlet 84 to allow mains or other water to pass from the inlet 82 into the mixing tank 4. A removable plug 76 allows access to a valve passage 92 within the valve housing 26 (Figure 13). A valve stem 86 is housed for axial motion within the valve passage 92. The valve stem 86 has a dome washer 88 at one end and a sealing head 106 at the other end with a projection 90, for releasable engagement with the distal end 58 of the actuator 54. A valve seat 94 in the valve passage 92 is sealable by the dome washer 88 when the valve stem 86 is moved to the closed position shown in Figures 11 and 13. The valve stem 86 is urged to the closed position by a spring 104 within a wider-bore portion 108 of the valve passage 92.

When the actuator 54 is in the first (STANDBY) position shown in Figures 11 and 13, the button 10 in this example is disposed wholly or partly outside the top cover 6. In this position, the drain control arm 66 is located in the first notch 60 and the washer 68 is separated from the drain outlet flange 70. Any liquid in the tank 4 will drain out through the outlet 8. The dose arm unit 38 is located in the second notch 62. At this, its lowest point, the dose arm unit 38 maintains the shuttle valves 46 in sealing engagement with the outlets 34 of the reservoirs 32. In this position, the shuttle valves 46 do not block the passage of liquids from the cartridges 12 to the reservoirs 32, which each become filled with a reagent liquid. In the STANDBY position, the dome washer 88 seals the valve seat 94, preventing water passing into the mixing tank 4 from the inlet 82.

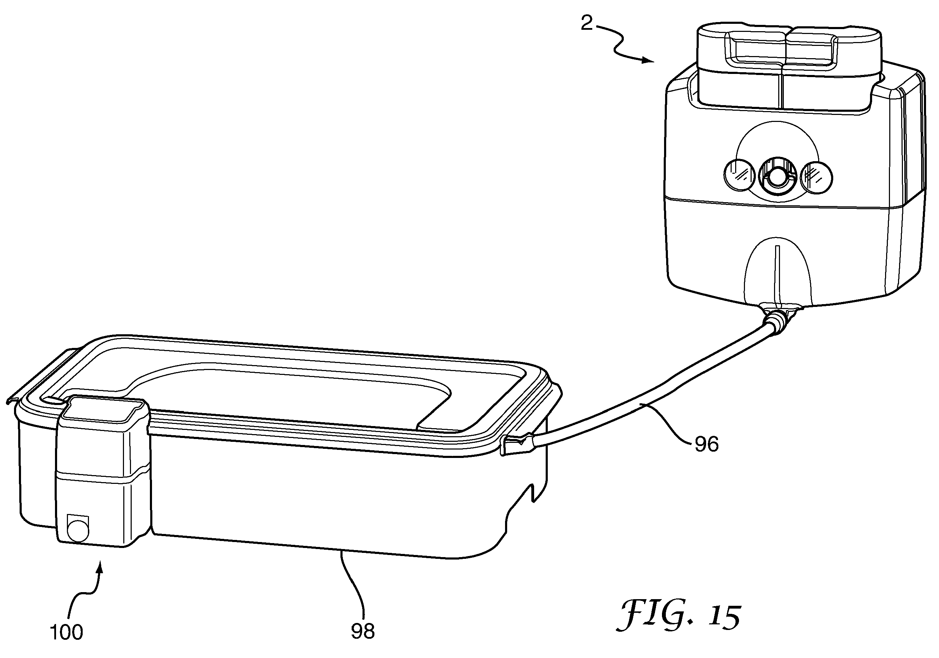

To prepare and dispense a predetermined volume of sterilizing liquid, a user first checks by looking through the windows 14 that each reservoir 32 is full of liquid and ascertains that a receptacle is in place to receive the sterilizing liquid from the outlet 8. The receptacle could be a simple bucket, jug or tray. In the example illustrated in Figure 15, the receptacle is a tray 98 for sterilizing medical instruments such as endoscopes. The tray 98 in this example is provided with an electronic controller 100 for automatic draining of sterilant after a predetermined sterilizing time, as described in WO 2008/020770. In this example, the outlet 8 is fitted with a standard CPC connector which connects the tank 4 to the tray 98 via a flexible tube 96.

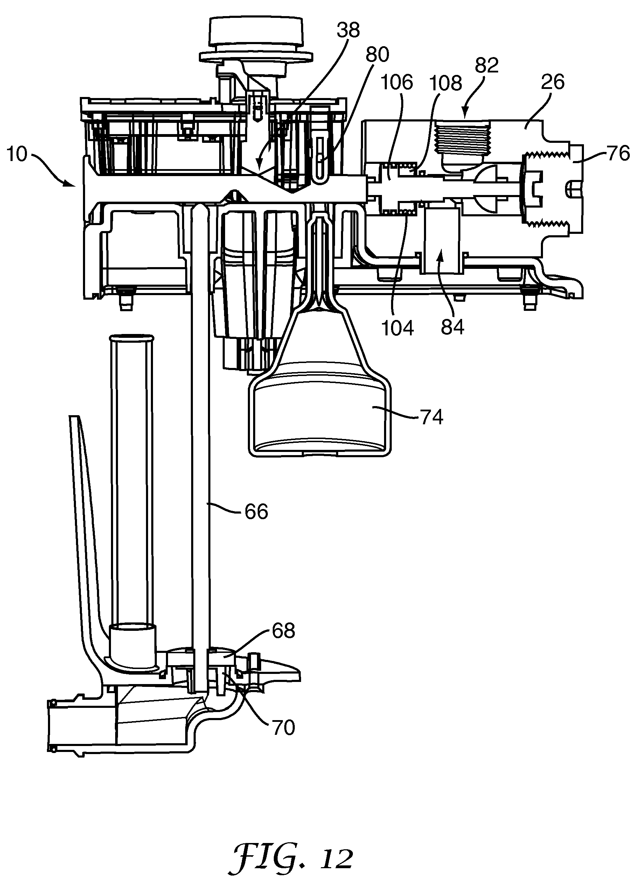

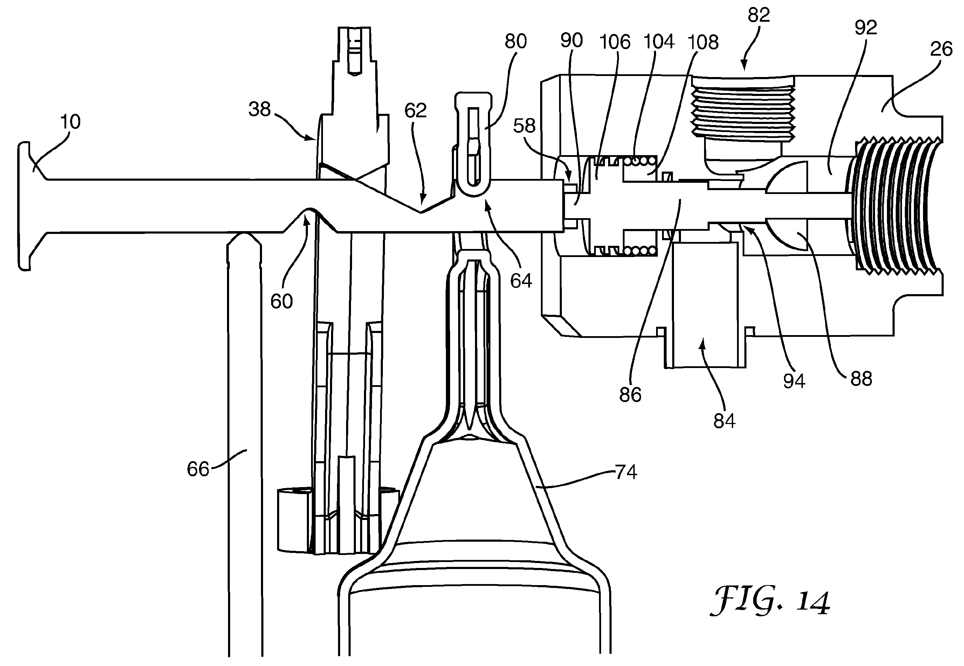

After verifying that the reservoirs 32 are full of liquid and that a suitable receptacle is in place to receive the sterilizing liquid, the user initiates preparation and dispensing of the sterilizing liquid by pushing the button 10 so as to drive the actuator 54 fully to the right as illustrated in Figures 12 and 14.

With the actuator 54 in this second (ACTIVE) position, the following changes occur:
- the drain control arm 66 is disengaged from the first notch 60 and pushed downwards by the shaft 56, causing the washer 68 to move into sealing engagement with the drain outlet flange 70, thereby preventing direct fluid communication between the inside of the tank 4 and the outlet 8 and maintaining the outlet 8 in a closed state;
- the dose arm unit 38 is disengaged from the second notch 62 and pushed upwards by the shaft 56. This movement causes the shuttle valves 46 to lift from the outlets 34 in the reservoirs 32 and to block the passage of liquids from the cartridges 12 to the reservoirs 32. The reagent liquids drain from the reservoirs 32 into the tank via the outlets 34;
- the shut-off float arm 80 drops into the groove 64 which captures it and prevents further axial movement of the shaft 56;
- the distal end of the actuator 54 engages with the projection 90 on the inlet valve stem 86 and axially pushes the valve stem 86, compressing the spring 104 and causing the dome washer 88 to move out of sealing engagement with the valve seat 94; this allows mains water to pass from the inlet 82 through the valve passage 92 to the mixing tank 4 via the water outlet 84.

The water and the reagent liquids mix within the mixing tank 4. When the liquid level in the tank 4 reaches a predetermined value, for example five litres, the shut-off float 74 is buoyed up by the liquid and rises sufficiently to allow the shut-off float arm 80 to be lifted from the groove 54 in the shaft 56. The spring 104 acts as a biasing means and urges the actuator 54 back to the STANDBY position shown in Figures 11 and 13.

As described previously, in the STANDBY mode, the drain control arm 66 is lifted, maintaining the outlet 8 in an open state allowing sterilizing liquid to drain out automatically through the outlet 8. The dose arm unit 38 is returned to the second notch 62, causing the shuttle valves 46 to seal off the outlets 34 of the reservoirs 32 and permit the draining of reagent liquids from the cartridges 12 to the reservoirs 32. The dome washer 88 seals against the valve seat 94, thereby shutting off the supply of water to the tank 4.

After the sterilizing liquid has drained from the mixing tank 4, the dispenser is ready for another batch of sterilizing liquid to be prepared and dispensed by pressing of the button 10.

In the described embodiment, no external power supply is required, only a source of mains water. However it will be appreciated that other embodiments may employ electrical power for one or more functions. Such power may be provided by mains or battery. For example, inductive or other sensors may be employed to detect fluid levels in the reservoirs 32 and/or in the mixing tank 4. The actuator 54 may operate a solenoid to maintain the valve controlling water input to the mixing tank in an open state until the solenoid is switched off in response to a signal that the liquid level in the mixing tank has reached a predetermined level. In other embodiments, some or all functions of the electronic controller 100 could optionally be provided within the sterilizing liquid dispenser 2.

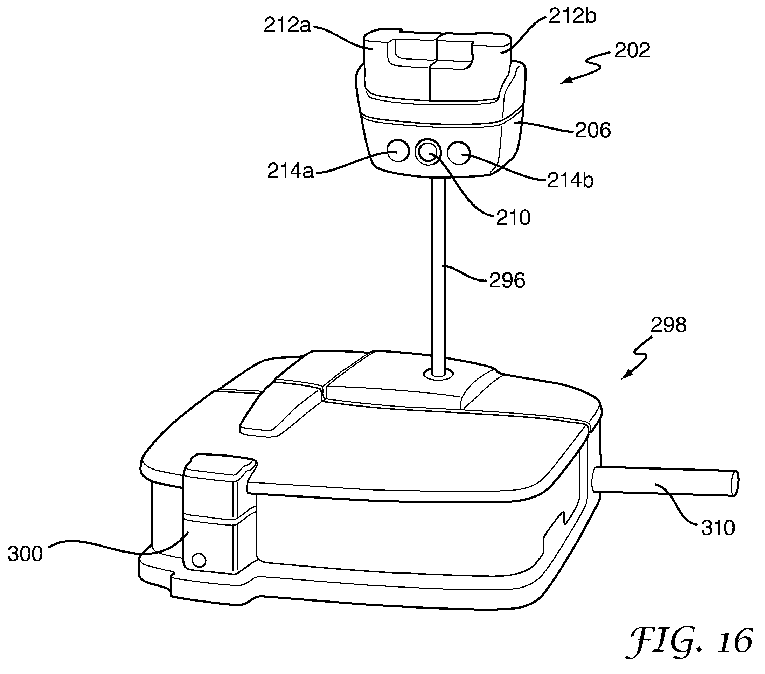

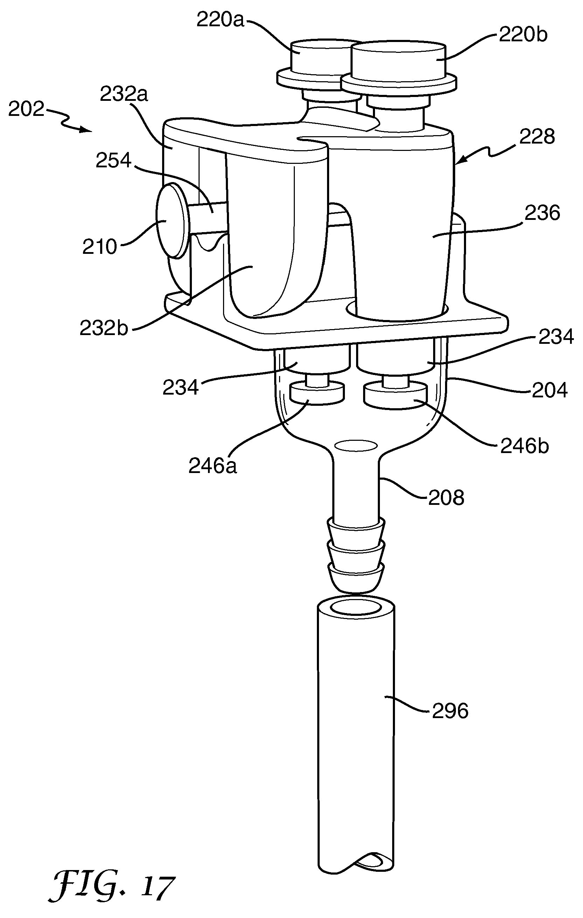

A second exemplified sterilizing liquid dispenser 202 is shown in Figures 16 and 17. The dispenser 202 operates in a similar manner to that of the dispenser 2 of the first embodiment, and like features have been indicated by reference numerals incremented by 200.

In this example, the dispenser 202 comprises an actuating or dispensing mechanism for dispensing fluid reagents and a mixing tank 204, but does not include an inlet for a supply of water. The outlet 208 of the mixing tank 204 is connected via a tube or hose 296 to a sterilization container 298, which in this embodiment is in the form of a tray, for containing a sterilizing liquid. The tray is provided with an electronic controller 300 for automatic control of a number of functions of the tray 298 as explained further below.

The dispenser 202 comprises a reservoir unit 228 including first and second reservoirs 232 for receiving first and second fluid reagents. First and second reagent cartridges 212a, 212b are connectable to the reservoir unit 228 via respective cartridge couplings 220a, 220b, providing a fluid connection between the cartridges 220a, 220b and the reservoirs 232a, 232b.

In this embodiment, each reservoir 232a, 232b includes a corresponding integrally formed valve housing 236 having an outlet 234 in fluid connection to the mixing tank 4, and the cartridge couplings 220a, 220b are arranged to form inlets of the respective valve housings 236.

A reservoir valve, in this example a shuttle valve, 246a, 246b is housed in each corresponding housing 236. When the shuttle valves 246 are in a first position the outlets 234 are open and a fluid seal is formed between the cartridge coupling 20 and the reservoir 32. In this configuration, reagent is prevented from flowing from the cartridges 212 into the reservoirs 232, but reagent already present in the reservoirs 232 will drain through the outlet 234 into the mixing tank 4. When the shuttle valves 246 are in a second position the reservoir outlets 234 are sealed, preventing flow of liquid from the reservoirs 232 into the mixing tank 4, and the inlets are open, permitting a flow of reagent from the cartridges 212 into the reservoirs 232 via the cartridge couplings 220.

The reservoir unit 228 is housed within a cover 206 including viewing windows 214a, 214b. Preferably, at least a part of the reservoir unit 228 is formed from a translucent plastics material so that a user can view fluid levels in each of the reservoirs 232 through the windows 214a, 214b in the cover 206.

The shuttle valves 246 are operated by means of an actuator 254, in the form of a push-rod having a button 210 at one end. A dose arm unit (not shown) engages with the shuttle valves 246 and the actuator 254 as described above in relation to the first embodiment. In particular, the actuator 254 comprises a notch providing a cam surface for engagement with a corresponding surface on the dose arm unit.

The actuator 254 is engaged with a biasing means (not shown) which is preferably in the form of a compression spring. The spring is arranged to bias or urge the actuator 254 into a first, STANDBY, position in which the dose arm unit is seated in the notch and the shuttle valves are in the second position, in which the reservoirs outlets 234 are sealed.

To operate the dispenser 202, a user presses the button 210 to move the actuator 254 against the action of the spring to a second, ACTIVE, position. In this second position, the dose arm unit is disengaged from the notch and movement of the dose arm unit moves the shuttle valves into the first position, in which the reservoir outlets are opened. In this second position, therefore, the reagent liquids drain from the reservoirs 232 into the mixing tank 204 via the outlets 234.

Preferably the actuator 254 and biasing means are arranged such that once a user has pressed and released the button 210 to dispense the reagent liquids, the biasing means urges the actuator back to the first, STANDBY, position. The time taken for the actuator 254 to return to the first position should be sufficient for the reagent liquids to fully drain from the reservoirs 232 before the outlet 234 of the reservoir is sealed.

In some embodiments the actuating mechanism may comprises latching means to delay the return of the actuator 254 from the second position to the first position.

In this embodiment, the outlet 208 of the mixing tank 204 is permanently open and does not include valve means. In this way, when the reagent liquids enter the mixing tank 204 via the outlets 234 they pass through the mixing tank 204 and then flow, unimpeded, out of the tank 204 via the outlet 208 under gravity. The mixing tank 204 is, preferably, shaped to provide some mixing of the reagent liquids before they exit the tank 204 through the outlet 208.

The reagent liquids, which in general will be at least partially mixed, flow through the tube 296 into the tray 298. The tray 298 also includes an inlet connected to a suitable supply of water by an inlet tube 310. The inlet preferably includes an inlet valve that is controlled by the electronic controller 300. When the inlet valve is open water is able to flow into the tray 298. A liquid level sensor within the tray 298 detects the level of the liquid within the tray 298 and outputs a signal to the controller 300. When the liquid in the tray 298 reaches a desired level, the controller 300 sends a signal to close the inlet valve and prevent additional water from flowing into the tray 298.

In this example the tray 298 is designed to enable sterilization of medical equipment within the tray and the electronic controller 300 enables automatic draining of sterilant from the tray 298 after a predetermined sterilizing time. It will be understood that the sterilization container may be used and sold separately from the sterilization dispenser. Accordingly, another aspect of the invention provides a sterilization container for receiving an item to be sterilized;
the sterilization container having a valve-controlled inlet for a water supply, a valve-controlled outlet, a liquid-level sensor, and an electronic controller arranged and adapted to close the inlet when the liquid-level sensor detects that the container has been filled with liquid to a predetermined level.

In other embodiments, the outlet tube 296 from the dispenser 202 may dispense reagent liquids into any other suitable container, such as a jug or bucket, which may be pre-filled with a required volume of water.

Although the dispenser 202 of this embodiment included a mixing tank 204, an outlet 208 and an outlet tube 296, in other embodiments the dispenser may not include these features. In these embodiments, the reagent liquids may be dispensed directly from the outlets 234 of the reservoirs 232 into a suitable receptacle without pre-mixing within the dispenser.

The invention has been exemplified with reference to the use of two reagent liquids which react when mixed to provide aqueous ClO₂ sterilant; for example a chlorite and an acid. The invention is not limited to particular reagents, sterilants, or chemistries and it will be understood that any suitable alternative reagents known to those skilled in the art may be used in the present invention. It will also be appreciated that more than two fluids may be dispensed and mixed with the water by using one or more additional cartridges and reservoirs and corresponding components operated by adjustment of the actuator. The preferred sterilising agent, chlorine dioxide, may be formed from suitable known reagents. In a preferred embodiment one reagent is a chlorite (notably sodium chlorite) and the other is an acid, preferably with a buffer. Suitable acids include lactic acid, citric acid, boric acid, phosphoric acid, acetic acid, sorbic acid, ascorbic acid, hydrochloric acid or mixtures thereof. In a preferred embodiment a mixture of acids is used, notably a mixture of citric, sorbic and boric acids.

Other sterilising agents may also be employed; for example chlorine or oxygen. Chlorine may be produced by reaction between a hypochlorite such as sodium hypochlorite, and a suitable acid or buffer. Oxygen may be produced by reaction between a peroxide and a catalyst such as catalase, optionally in the presence of a buffer.

It is to be recognized that various alterations, modifications, and/or additions may be introduced into the constructions and arrangements of parts described above without departing from the ambit of the present invention as specified in the accompanying claims.

## Claims

1. A sterilizing liquid dispenser for dispensing a measured amount of at least two fluid reagents, the dispenser comprising:
- a first reservoir for a first fluid reagent, the reservoir having a first reservoir inlet valve and a first reservoir outlet valve;
- a second reservoir for a second fluid reagent, the reservoir having a second reservoir inlet valve and a second reservoir outlet valve; and
- a user-operable actuator;
wherein the actuator is operable to:
- close the first and second reservoir inlet valves, to prevent fluid reagent entering the first and second reservoirs; and
- open the first and second reservoir outlet valves, to permit dispensing of the first and second fluid reagents from the reservoirs.

2. A dispenser according to Claim 1, further comprising a mixing tank having an outlet, wherein the mixing tank is arranged such that the first and second reagents can be dispensed from the reservoirs into the mixing tank and the outlet permits a mixture of the first and second reagents to be dispensed from the mixing tank.

3. A dispenser according to Claim 2, wherein the mixing tank comprises a valve-controlled outlet and the dispenser further comprises means for opening the valve-controlled outlet to permit dispensing of the mixture of reagents from the mixing tank.

4. A dispenser according to Claim 3, wherein the means for opening the valve-controlled outlet comprises means for automatically opening the valve-controlled outlet when liquid within the mixing tank reaches a predetermined level.

5. A dispenser according to any of Claims 2 to 4, wherein the mixing tank comprises a valve-controlled inlet for water and the actuator is arranged to open the valve controlled inlet to permit said water to enter the mixing tank.

6. A dispenser according to Claim 5, further comprising means for automatically closing the valve-controlled inlet to prevent water from entering the tank when liquid in the tank reaches a predetermined level.

7. A dispenser according to any one of Claims 2 to 6, further comprising means for simultaneously closing the first and second reservoir outlet valves to seal off the first reservoir and the second reservoir from the mixing tank when liquid in the tank reaches a predetermined level.

8. A dispenser according to any preceding claim, wherein the actuator comprises a push-rod movable, by a user, from a first position to a second position, and wherein in the first position the first and second reservoir inlet valves are open and the first and second reservoir outlet valves are closed and in the second position the first and second reservoir inlet valves are closed and the first and second reservoir outlet valves are open.

9. A dispenser according to Claim 8, when dependent on Claim 3, wherein in the first position the valve-controlled outlet of the mixing tank is open and in the second position the valve-controlled outlet of the mixing tank is closed.

10. A dispenser according to Claim 8 or Claim 9, when dependent on Claim 5, wherein in the first position the valve-controlled inlet for water is closed and in the second position the valve-controlled inlet for water is open.

11. A dispenser as claimed in any of Claims 8 to 10, wherein the push-rod is engaged with biasing means, the biasing means being arranged to bias the push-rod in the first position.

12. A dispenser as claimed in Claim 11 when dependent on Claim 10, wherein the push-rod has a proximal end and a distal end and wherein sufficient pushing of the proximal end by a user will move the push-rod from the first position to the second position against the force of the biasing means and in the second position the distal end will push a valve member of the valve-controlled inlet for water to open the inlet.

13. A dispenser according to Claim 12, further comprising a float member with a float arm; wherein the push-rod is provided with a feature with which the float arm will engage when the distal end of the push-rod has travelled a predetermined distance, the engagement substantially preventing further travel of the push-rod until the float member has been buoyed to a predetermined level by liquid within the mixing tank.

14. A dispenser according to Claim 9 or any of Claims 10 to 13 when dependent on Claim 9, wherein the valve-controlled outlet of the mixing tank is operated by movement of a drain control arm; the push-rod and the drain control arm having interengageable cam surfaces arranged and constructed so that when the push-rod is in the first position the drain control arm maintains the outlet in an open state, and when the push-rod is in the second position the drain control arm maintains the outlet in a closed state.

15. A dispenser according to any one of Claims 8-14, wherein each reservoir outlet valve is operated by movement of a dose arm unit and wherein the push-rod and the dose arm unit have interengageable cam surfaces arranged and constructed so that when the push-rod is in the first position the dose arm maintains the reservoir outlet valves in a closed position and when the push-rod is in the second position the dose arm maintains the reservoir outlet valves in an open position.

16. A dispenser according to any preceding claim, wherein each reservoir has a cartridge port for fluid connection to a reagent cartridge; and wherein when the reservoir inlet valves are in the open position each cartridge port is unsealed permitting fluid to flow from the reagent cartridge into the reservoir, and when the reservoir inlet valves are in the closed position each cartridge port is sealed preventing fluid from flowing from the reagent cartridge into the reservoir.

17. Sterilizing apparatus comprising:
a sterilizing liquid dispenser according to any preceding claim, and
a sterilization container for receiving an item to be sterilized and arranged to receive sterilizing fluid from the dispenser;
the sterilization container having a valve-controlled inlet for a water supply, a valve-controlled outlet, a liquid-level sensor, and an electronic controller arranged and adapted to close the inlet when the liquid-level sensor detects that the container has been filled with liquid to a predetermined level.

## Patentansprüche

1. Sterilflüssigkeitsspender zum Verspenden einer abgemessen Menge von wenigstens zwei flüssigen Reagenzien, wobei der Spender Folgendes umfasst:
- ein erstes Reservoir für eine erste flüssige Reagenz, wobei das Reservoir ein erstes Reservoireinlass-ventil und ein erstes Reservoirauslassventil aufweist;
- ein zweites Reservoir für eine zweite flüssige Reagenz, wobei das Reservoir ein zweites Reservoireinlass-ventil und ein zweites Reservoirauslassventil aufweist; und
- einen durch einen Benutzer zu betätigenden Aktuator;
wobei der Aktuator betätigt werden kann, um
- das erste und das zweite Reservoireinlassventil zu schließen, um zu verhindern, dass eine flüssige Reagenz in das erste und das zweite Reservoir eintritt; und
- das erste und das zweite Reservoirauslassventil zu öffnen, um das Verspenden der ersten oder zweiten flüssigen Reagenz aus den Reservoiren zu ermöglichen.

2. Spender nach Anspruch 1,
welcher weiterhin einen Mischtank mit einem Auslass umfasst, wobei der Mischtank so angeordnet ist, dass die erste und zweite Reagenz aus den Reservoiren in den Mischtank verspendet werden können, und der Auslass ermöglicht, dass eine Mischung aus der ersten und zweiten Reagenz aus dem Mischtank verspendet wird.

3. Spender nach Anspruch 2,
bei dem der Mischtank einen ventilgesteuerten Auslass umfasst und der Spender weiter ein Mittel zum Öffnen des ventilgesteuerten Auslasses umfasst, um das Verspenden der Mischung aus Reagenzien aus dem Mischtank zu ermöglichen.

4. Spender nach Anspruch 3,
bei dem das Mittel zum Öffnen des ventilgesteuerten Auslasses ein Mittel zum automatischen Öffnen des ventilgesteuerten Auslasses umfasst, wenn die Flüssigkeit innerhalb des Mischtanks ein vorgegebenes Niveau erreicht.

5. Spender nach einem der Ansprüche 2 - 4,
bei welchem der Mischtank einen ventilgesteuerten Einlass für Wasser umfasst und der Aktuator so angeordnet ist, dass er den ventilgesteuerten Einlass öffnen kann, um zu ermöglichen, dass das Wasser in den Mischtank eintritt.

6. Spender nach Anspruch 5,
weiter umfassend ein Mittel zum automatischen Schließen des ventilgesteuerten Einlasses, um zu verhindern, dass Wasser in den Tank eintritt, wenn die Flüssigkeit in dem Tank ein vorgegebenes Niveau erreicht.

7. Spender nach einem der Ansprüche 2 - 6,
weiter umfassend ein Mittel zum gleichzeitigen Schließen des ersten und zweiten Reservoirauslassventils, um das erste Reservoir und das zweite Reservoir von dem Mischtank zu trennen, wenn die Flüssigkeit im Tank ein vorgegebenes Niveau erreicht.

8. Spender nach einem der vorigen Ansprüche,
bei welchem der Aktuator eine durch einen Benutzer aus einer ersten Position in eine zweite Position bewegbare Schubstange umfasst, und wobei in der ersten Position das erste und das zweite Reservoireinlassventil geöffnet und das erste und das zweite Reservoirauslassventil geschlossen sind und in der zweiten Position das erste und das zweite Reservoireinlassventil geschlossen und das erste und das zweite Reservoirauslassventil geöffnet sind.

9. Spender nach Anspruch 8, soweit von Anspruch 3 abhängig,
bei welchem in der ersten Position der ventilgesteuerte Auslass des Mischtanks offen und in der zweiten Position der ventilgesteuerte Auslass des Mischtanks geschlossen ist.

10. Spender nach Anspruch 8 oder Anspruch 9, soweit von Anspruch 5 abhängig,
bei welchem in der ersten Position der ventilgesteuerte Einlass für Wasser geschlossen und in der zweiten Position der ventilgesteuerte Einlass für Wasser geöffnet ist.

11. Spender nach einem der Ansprüche 8 - 10,
bei dem die Schubstange mit einem Vorspannmittel zusammenwirkt, wobei das Vorspannmittel so angeordnet ist, dass es die Schubstange in der ersten Position vorspannt.

12. Spender nach Anspruch 11, soweit von Anspruch 10 abhängig,
bei welchem die Schubstange ein nahes Ende und ein entferntes Ende aufweist, und wobei ein hinreichendes Schieben des nahen Endes durch einen Benutzer die Schubstange aus der ersten Position in die zweite Position gegen die Kraft des Vorspannmittels bewegt und wobei in der zweiten Position das entfernte Ende ein Ventilelement des ventilgesteuerten Einlasses für Wasser verschiebt, um den Einlass zu öffnen.

13. Spender nach Anspruch 12,
weiter umfassend ein Schwimmelement mit einem Schwimmarm; wobei die Schubstange mit einer Einrichtung versehen ist, mit welcher der Schwimmarm zusammengreift, wenn das entfernte Ende der Schubstange sich um einen vorgegebenen Abstand bewegt hat, wobei der Eingriff im Wesentlichen das weitere Verschieben der Schubstange so lange verhindert, bis das Schwimmelement bis zu einem vorgegebenen Niveau durch das Wasser im Mischtank angehoben worden ist.

14. Spender nach Anspruch 9 oder einem der Ansprüche 10 - 13, sofern von Anspruch 9 abhängig,
bei welchem der ventilgesteuerte Auslass des Mischtanks durch die Bewegung eines Ablaufsteuerarms betätigt wird; wobei die Schubstange und der Ablaufsteuerarm miteinander zusammenwirkende Nockenflächen aufweisen, die so angeordnet und ausgebildet sind, dass wenn die Schubstange sich in der ersten Position befindet, der Ablaufsteuerarm den Auslass in einem offenen Zustand hält, und wenn die Schubstange sich in der zweiten Position befindet, der Ablaufsteuerarm den Auslass in einem geschlossenen Zustand hält.

15. Spender nach einem der Ansprüche 8 - 14,
bei welchem jedes Reservoirauslassventil durch die Bewegung einer Dosierarmeinheit betätigt wird, und wobei die Schubstange und die Dosierarmeinheit miteinander zusammenwirkende Nockenflächen aufweisen, welche so angeordnet und ausgebildet sind, dass wenn die Schubstange sich in der ersten Position befindet, der Dosierarm die Reservoirauslassventile in einer geschlossenen Position hält, und wenn die Schubstange sich in der zweiten Position befindet, der Dosierarm die Reservoiraus-lassventile in einer offenen Position hält.

16. Spender nach einem der vorigen Ansprüche,
bei welchem jedes Reservoir einen Kartuschenanschluss zur Fluidverbindung mit einer Reagenzienkartusche aufweist; und wobei, wenn die Reservoireinlassventile sich in der geöffneten Position befinden, jeder Kartuschen-anschluss geöffnet wird, um zu ermöglichen, dass Flüssigkeit aus der Reagenzienkartusche in das Reservoir fließt, und wenn die Reservoireinlassventile sich in der geschlossenen Position befinden, jeder Kartuschen-anschluss abgedichtet wird, um zu verhindern, dass Flüssigkeit aus der Reagenzienkartusche in das Reservoir fließt.

17. Sterilisierungsvorrichtung, umfassend:
einen Sterilisationsflüssigkeitsspender nach einem der vorigen Ansprüche, und
einen Sterilisationsbehälter, der zur Aufnahme eines zu sterilisierenden Gegenstandes dient und der außerdem so hergerichtet ist, dass er Sterilisierungsflüssigkeit aus dem Spender aufnehmen kann;
wobei der Sterilisationsbehälter einen ventilgesteuerten Einlass für die Wasserzufuhr, einen ventilgesteuerten Auslass, einen Flüssigkeitsspiegelsensor und eine elektronische Steuerung aufweist, welche so angeordnet und ausgelegt ist, dass sie den Einlass verschließt, wenn der Flüssigkeitsspiegelsensor detektiert, dass der Container bis zu einem vorgegebenen Niveau mit Flüssigkeit gefüllt ist.

## Revendications

1. Distributeur de liquide de stérilisation pour distribuer une quantité mesurée d'au moins deux réactifs fluides, le distributeur comprenant :
- un premier réservoir pour un premier réactif fluide, le réservoir ayant une valve d'entrée de premier réservoir et une valve de sortie de premier réservoir ;
- un second réservoir pour un second réactif fluide, le réservoir ayant une valve d'entrée de second réservoir et une valve de sortie de second réservoir ; et
- un actionneur actionnable par utilisateur ;
l'actionneur étant actionnable pour :
- fermer les valves d'entrée des premier et second réservoirs, pour empêcher le réactif fluide d'entrer dans les premier et second réservoirs ; et
- ouvrir les valves de sortie des premier et second réservoirs, pour permettre la distribution des premier et second réactifs fluides à partir des réservoirs.

2. Distributeur selon la revendication 1, comprenant en outre une cuve de mélange ayant une sortie, la cuve de mélange étant agencée de telle sorte que les premier et second réactifs peuvent être distribués à partir des réservoirs dans la cuve de mélange et la sortie permet à un mélange des premier et second réactifs d'être distribué à partir de la cuve de mélange.

3. Distributeur selon la revendication 2, dans lequel la cuve de mélange comprend une sortie commandée par valve et le distributeur comprend en outre des moyens pour ouvrir la sortie commandée par valve pour permettre la distribution du mélange de réactifs à partir de la cuve de mélange.

4. Distributeur selon la revendication 3, dans lequel les moyens pour ouvrir la sortie commandée par valve comprennent des moyens pour ouvrir automatiquement la sortie commandée par valve lorsque du liquide à l'intérieur de la cuve de mélange atteint un niveau prédéterminé.

5. Distributeur selon l'une quelconque des revendications 2 à 4, dans lequel la cuve de mélange comprend une entrée commandée par valve pour de l'eau et l'actionneur est agencé pour ouvrir l'entrée commandée par valve pour permettre à ladite eau d'entrer dans la cuve de mélange.

6. Distributeur selon la revendication 5, comprenant en outre des moyens pour fermer automatiquement l'entrée commandée par valve pour empêcher de l'eau d'entrer dans la cuve lorsque le liquide dans la cuve atteint un niveau prédéterminé.

7. Distributeur selon l'une quelconque des revendications 2 à 6, comprenant en outre des moyens pour fermer simultanément les valves de sortie des premier et second réservoirs pour sceller le premier réservoir et le second réservoir vis-à-vis de la cuve de mélange lorsque du liquide dans la cuve atteint un niveau prédéterminé.

8. Distributeur selon l'une quelconque des revendications précédentes, dans lequel l'actionneur comprend une tige-poussoir mobile, par un utilisateur, d'une première position à une seconde position, et dans lequel, dans la première position, les valves d'entrée des premier et second réservoirs sont ouvertes et les valves de sortie des premier et second réservoirs sont fermées et, dans la seconde position, les valves d'entrée des premier et second réservoirs sont fermées et les valves de sortie des premier et second réservoirs sont ouvertes.

9. Distributeur selon la revendication 8 prise en dépendance de la revendication 3, dans lequel, dans la première position, la sortie commandée par valve de la cuve de mélange est ouverte et, dans la seconde position, la sortie commandée par valve de la cuve de mélange est fermée.

10. Distributeur selon la revendication 8 ou la revendication 9, prise en dépendance de la revendication 5, dans lequel, dans la première position, l'entrée commandée par valve pour l'eau est fermée et, dans la seconde position, l'entrée commandée par valve pour l'eau est ouverte.

11. Distributeur selon l'une quelconque des revendications 8 à 10, dans lequel la tige-poussoir est engagée avec des moyens de sollicitation, les moyens de sollicitation étant agencés pour solliciter la tige-poussoir dans la première position.

12. Distributeur selon la revendication 11, prise en dépendance de la revendication 10, dans lequel la tige-poussoir a une extrémité proximale et une extrémité distale et dans lequel une poussée suffisante de l'extrémité proximale par un utilisateur déplacera la tige-poussoir de la première position à la seconde position à l'encontre de la force des moyens de sollicitation et, dans la seconde position, l'extrémité distale poussera un élément de valve de l'entrée commandée par valve pour l'eau pour ouvrir l'entrée.

13. Distributeur selon la revendication 12, comprenant en outre un élément flotteur avec un bras de flotteur ; dans lequel la tige-poussoir comprend une caractéristique avec laquelle le bras de flotteur sera engagé lorsque l'extrémité distale de la tige-poussoir aura parcouru une distance prédéterminée, l'engagement empêchant sensiblement un autre déplacement de la tige-poussoir jusqu'à ce que l'élément flotteur soit remonté à un niveau prédéterminé par le liquide à l'intérieur de la cuve de mélange.

14. Distributeur selon la revendication 9 ou l'une quelconque des revendications 10 à 13 prises en dépendances de la revendication 9, dans lequel la sortie commandée par valve de la cuve de mélange est actionnée par mouvement d'un bras de commande d'évacuation ; la tige-poussoir et le bras de commande d'évacuation ayant des surfaces de came engageables réciproquement agencées et construites de telle sorte que, lorsque la tige-poussoir est dans la première position, le bras de commande d'évacuation maintient la sortie dans un état ouvert et, lorsque la tige-poussoir est dans la seconde position, le bras de commande d'évacuation maintient la sortie dans un état fermé.

15. Distributeur selon l'une quelconque des revendications 8 à 14, dans lequel chaque valve de sortie de réservoir est actionnée par mouvement d'une unité de bras de dosage et dans lequel la tige-poussoir et l'unité de bras de dosage ont des surfaces de came engageables réciproquement agencées et construites de telle sorte que, lorsque la tige-poussoir est dans la première position, le bras de dosage maintient les valves de sortie de réservoir dans une position fermée et, lorsque la tige-poussoir est dans la seconde position, le bras de dosage maintient les valves de sortie de réservoir dans une position ouverte.

16. Distributeur selon l'une quelconque des revendications précédentes, dans lequel chaque réservoir a un orifice de cartouche pour raccordement fluidique à une cartouche de réactif ; et dans lequel, lorsque les valves d'entrée de réservoir sont dans la position ouverte, chaque orifice de cartouche est descellé pour permettre au fluide de s'écouler depuis la cartouche de réactif dans le réservoir et, lorsque les valves d'entrée de réservoir sont dans la position fermée, chaque orifice de cartouche est scellé pour empêcher le fluide de s'écouler depuis la cartouche de réactif dans le réservoir.

17. Appareil de stérilisation comprenant :
un distributeur de liquide de stérilisation selon l'une quelconque des revendications précédentes, et
un récipient de stérilisation pour recevoir un article à stériliser et agencé pour recevoir un fluide de stérilisation à partir du distributeur ;
le récipient de stérilisation ayant une entrée commandée par valve pour une alimentation en eau, une sortie commandée par valve, un capteur de niveau de liquide, et une unité de commande électronique agencée et adaptée pour fermer l'entrée lorsque le capteur de niveau de liquide détecte que le récipient a été rempli de liquide à un niveau prédéterminé.
